# EUROPÄISCHE PATENTSCHRIFT

(11) **EP 1 817 274 B1**
(45) Veröffentlichungstag und Bekanntmachung des Hinweises auf die Patenterteilung: **18.03.2009**
(21) Anmeldenummer: 05816199.3
(22) Anmeldetag: 29.11.2005
(51) Int. Cl.: C07C 253/22, C07C 253/34, C07C 255/53

(54) **VERFAHREN ZUR HERSTELLUNG VON AMINO- ODER HYDROXYBENZONITRILEN**
METHOD FOR PRODUCING AMINO- OR HYDROXYBENZONITRILES
PROCEDE DE PRODUCTION D'AMINOBENZONITRILES OU D'HYDROXYBENZONITRILES

(30) Priorität: 01.12.2004 DE 102004058001; 15.11.2005 DE 102005054362
(43) Veröffentlichungstag der Anmeldung: 15.08.2007
(73) Patentinhaber: AlzChem Trostberg GmbH, 83308 Trostberg (DE)
(72) Erfinder: MÖLLER, Roland, 63546 Hammersbach (DE); GOMEZ, Mario, 64319 Pfungstadt (DE); EINMAYR, Klaus, 83342 Tacherting (DE); HILDEBRAND, Jens, 31141 Hildesheim (DE); ERBEN, Hans-Georg, 83071 Stephanskirchen (DE); KRIMMER, Hans-Peter, 84558 Kirchweidach (DE)
(74) Vertreter: Böhm, Brigitte
(86) Internationale Anmeldenummer: PCT/EP2005/012749
(87) Internationale Veröffentlichungsnummer: WO 2006/058710

(56) Entgegenhaltungen:
- WO-A-03/097582
- DE-A1- 19 520 491
- GB-A- 1 220 386
- PATENT ABSTRACTS OF JAPAN Bd. 013, Nr. 049 (C-565), 3. Februar 1989 (1989-02-03) & JP 63 243064 A (MITSUI TOATSU CHEM INC), 7. Oktober 1988 (1988-10-07)

## Beschreibung

Gegenstand der vorliegenden Erfindung ist ein neues Verfahren zur Herstellung von Amino- oder Hydroxybenzonitrilen, bei dem ein Herstellungsschritt 1) mit einem Aufreinigungsschritt 2) kombiniert wird.

Amino- und Hydroxybenzonitrile sind Produkte, an denen zunehmend größeres Interesse besteht, wobei insbesondere Hydroxybenzonitrile als Zwischenprodukte bei der Herstellung von Pflanzen-aktiven Wirkstoffen Verwendung finden.

Bei der Herstellung aromatischer Nitrile gibt es zahlreiche unterschiedliche Varianten, wobei hinsichtlich der Amino- oder Hydroxybenzonitrile die Ammonoxidation von Methylarenen bislang im Vordergrund stand. Beispielhaft seinen hier die deutsche Offenlegungsschrift DE 20 20 866 und das französische Patent FR 2 332 261 genannt.

Üblicherweise erfolgt die Herstellung von Hydroxybenzonitrilen über die Aminierung eines Alkylhydroxybenzoats, der sich eine Dehydratisierungsreaktion anschließt. Der deutschen Offenlegungsschrift DE 20 20 866 A1 kann ein Verfahren zur Herstellung von 4-Hydroxybenzonitril entnommen werden, wobei die Umsetzung von Ammoniak und 4-Hydroxybenzoesäuremethylester in der Gasphase und in Gegenwart eines geträgerten Phosphorsäurekatalysators, also als typische Ammonolyse, durchgeführt wird.

Eine ähnliche Reaktion ist im Patent FR 2 332 261 beschrieben, bei dem allerdings im Gegensatz zum eben genannten Verfahren ein Katalysator eingesetzt wird, der aus Borphosphat besteht, welches mit einem Übergangsmetall dotiert ist. Ein wesentliches Problem bei diesem Reaktionstyp ist darin zu sehen, dass sich das daraus erhaltene Produkt, nämlich die Hydroxybenzonitril-Verbindungen beim Kondensieren verfestigen, wodurch die Produktabtrennung erschwert wird. Außerdem werden bei dieser Reaktionsweise Nebenprodukte gebildet, die bspw. aus einer Trimerisierung der erhaltenen Produkte zu s-Triazin hervorgehen, was insbesondere bei höheren Temperaturen erfolgt. Eine weitere Nebenreaktion läuft als Hydrolyse des erhaltenen 2-Hydroxybenzonitril zu 2-Hydroxybenzamid ab, was insbesondere durch Wassermengen beschleunigt wird, die im Verlauf der Herstellung des Nitrils freigesetzt werden. Verstärkt wird diese Hydrolyse durch die Anwesenheit von Ammoniak.

Gegenüber den vielfach verwendeten Festbettverfahren besitzen Wirbelschichtreaktionen den Vorteil, dass die dabei verwendeten Katalysatoren bei weitem standfester sind, da sie weniger zu Verbackungen und Kanalbildungen neigen. Diese Nachteile hinsichtlich der Katalysatoren können auch aus der zitierten deutschen Offenlegungsschrift und dem französischen Patent abgeleitet werden, da sie auf Katalysatoren zurückgreifen, die genau die beschriebenen negativen Eigenschaften aufweisen.

Aus diesem Grund wurde bspw. in der deutschen Offenlegungsschrift DE 195 20 491 A1 ein Verfahren zur Herstellung von Amino- oder Hydroxybenzonitrilen vorgeschlagen, welches auf Katalysatoren zurückgreift, die eine hohe Abriebsfestigkeit aufweisen und deshalb insbesondere im Wirbelschichtverfahren eingesetzt werden können. Die dort beschriebenen Katalysatoren weisen eine hohe Belastung auf, was eine hohe Raum-ZeitAusbeute und eine hochwirtschaftliche Herstellung der genannten Nitrile möglich macht. Des Weiteren zeichnen sich die in dieser deutschen Offenlegungsschrift beschriebenen Katalysatoren durch eine hohe Selektivität aus.

Bei den Katalysatoren gemäß DE 195 20 491 A1 handelt es sich um Borphosphat-Trägerkatalysatoren, die mit Übergangsmetall-Verbindungen der 5., 6., 12. oder 14. Gruppe des Periodensystems der Elemente oder Kombinationen davon dotiert wurden und deren spezifische Oberfläche mindestens 400 m²/g beträgt.

Zwar ließen sich mit Hilfe dieser Trägerkatalysatoren Ausbeuten und Selektivitäten bis 100 % erreichen und die Katalysatoren hielten zusätzlich einer Belastung bis zu 5 mol Ausgangsprodukt pro kg Katalysator und Stunde stand. Jedoch bereitet die Abtrennung der gewünschten Produkte nach wie vor Probleme.

So ist man verstärkt bemüht, Verfahrensverbesserungen vorzunehmen, die insbesondere die Abtrennung von Hydroxybenzonitrilen vereinfachen.

An dieser Stelle sei insbesondere die internationale Anmeldung WO 01/96 284 genannt, die ein Verfahren zur Abtrennung einer Verbindung des Hydroxybenzonitril-Typs beschreibt, wobei die Endprodukte mit Hilfe eines Amidierungs-/Dehydratisierungs-Verfahrens gewonnen werden. Im Vordergrund der dort angestellten Betrachtungen steht insbesondere das 2-Hydroxybenzonitril (2-Cyanophenol).

Die in dieser Anmeldung beschriebene Abtrennung geht von einem Rohprodukt aus, welches in Form eines Ammoniumsalzes aus einem gasförmigen Reaktionsstrom gewonnen wird. Der Hauptreinigungsschritt besteht deshalb auch darin, Ammoniumionen zu verdrängen, was vor allen Dingen mit einer physikalischen Behandlung des gasförmigen Reaktionsstroms erfolgt. Möglich ist allerdings auch die physikalische Behandlung des zuvor aus dem gasförmigen Reaktionsstrom durch Verflüssigung erhaltenen Feststoffes oder die Behandlung des in Lösung gebrachten Feststoffes. Ebenfalls vorgeschlagen wird eine chemische Behandlung an dem verflüssigten, gasförmigen Reaktionsstrom.

Auffallend bei allen bislang kommerziell durchgeführten Verfahren zur Herstellung von Amino- oder Hydroxybenzonitrilen ist die Tatsache, dass diese von Estern ausgehen oder aber im Falle von Säuren, diese Säuren zunächst zu Säureamiden umsetzen, bevor die Reaktion zu den Nitrilen erfolgt. Da Säuren als Ausgangsmaterialien insbesondere in der Gasphase bei den zumeist hohen Temperaturen zur Zersetzung neigen, ist man dazu übergegangen, deren Ester einzusetzen, was sich aber wiederum nachteilig auf die Produktreinheit aufgrund entstehender Alkohole und damit zusammenhängender aromatischer Nebenprodukte auswirkt.

Aus dem Stand der Technik und den damit verbundenen Nachteilen heraus hat sich für die vorliegende Erfindung die Aufgabe gestellt, ein Verfahren zur Herstellung von Amino- oder Hydroxybenzonitrilen der allgemeinen Formel (I) 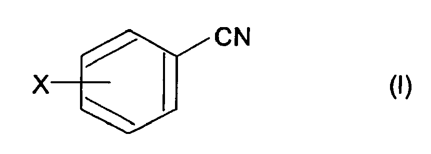 in der X für mindestens eine Amino- oder Hydroxygruppe steht, bereitzustellen, bei dem die Umsetzung einer Amino- oder Hydroxybenzoesäure-Verbindung der allgemeinen Formel (II) 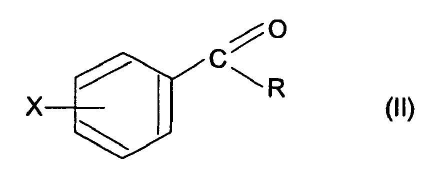 wobei R = -OH oder -NH₂ und X die oben genannte Bedeutung besitzt, mit Ammoniak in Gegenwart eines Phosphor-haltigen Trägerkatalysators bei Temperaturen zwischen 250 und 500 °C erfolgt. Durch die Wahl geeigneter Verfahrensschritte bzgl. der Herstellung sollte ein Rohprodukt erhalten werden, dessen Form die nachfolgende Aufreinigung in wirtschaftlicher Form gewährleistet, wobei das isolierte Endprodukt einen hohen Reinheitsgrad aufweisen sollte. Insbesondere sonst übliche Nebenprodukte sollten zurückgedrängt oder vollkommen beseitigt sein und sowohl das Herstellungsverfahren als auch das Aufreinigungsverfahren sollten unter Umweltgesichtspunkten Anforderungen genügen, die es zusätzlich unter wirtschaftlichen Aspekten attraktiv machen.

Gelöst wurde diese Aufgabe mit einem Verfahren, bei dem der Herstellungsschritt 1) in einem Reaktionsgas(-gemisch) und ohne Beteiligung eines organischen Lösemittels durchgeführt wird und bei dem sich ein mindestens zweistufiger Aufreinigungsschritt 2) anschließt, bei dem 2.1) das aus dem Herstellungsschritt 1) erhaltene gasförmige Gemisch oder feste Reaktionsprodukt in eine wässrige, ammoniumhaltige Suspension überführt wird und 2.2) a) die Freisetzung des im Feststoff als Ammoniumsalz vorliegenden Produktes mithilfe einer Filtration unter Säurezugabe oder durch Austreiben von Ammoniak und ein sich anschließendes Einleiten der Säurekomponente erfolgt, oder b) die Freisetzung des im Feststoff als Ammoniumsalz vorliegenden Produktes durch eine Reaktivdestillation vorgenommen wird, oder c) die Freisetzung des im Feststoff als Ammoniumsalz vorliegenden Produktes durch eine Abspaltung bei Temperaturen von 0 bis 100°C und in Gegenwart eines organischen Lösungsmittels durchgeführt wird.

Die Aufgabenstellung wurde insbesondere dadurch erfüllt, dass das erfindungsgemäße Verfahren bei weitem wirtschaftlicher und preisgünstiger durchzuführen ist als bspw. ähnliche Verfahren, bei denen zunächst das aus der entsprechenden Amino- oder Hydroxysäure erhaltene Säureamid mit Phosgen zum Nitril umgesetzt wird. Die aus diesem Verfahren in größeren Mengen anfallenden Salzkonzentrationen werden gänzlich vermieden bzw. stark reduziert, wobei die eingesetzten und nicht umgesetzten Säuren recycliert werden können. Hinzu kommt, dass mit dem erfindungsgemäßen Verfahren ein äußerst reines Produkt erhältlich ist, welches dramatisch reduzierte Phenol- und Alkoholgehalte als sonst typische Nebenprodukte aufweist.

Überraschend hat sich zusätzlich zur Erfüllung der Aufgabenstellung herausgestellt, dass mit dem erfindungsgemäßen Verfahren das bisher aus dem Stand der Technik bekannte Vorurteil, nämlich aromatische Nitrile in wirtschaftlicher Weise ausschließlich aus deren entsprechenden Säureestern herstellen zu können, erstmalig überwunden werden konnte. Wie sich nämlich gezeigt hat, können nicht nur die bislang aufgrund ihrer thermischen Stabilität und besseren Handhabbarkeit eingesetzten Ester verwendet werden. Wie die vorliegende Erfindung zeigt, können auch die aromatischen Säuren in aminierter und hydroxylierter Form als ebenfalls sehr gut geeignete Ausgangsverbindungen eingesetzt werden. Neben ihrem wirtschaftlichen Vorteil, der im deutlich niedrigeren Preis zu sehen ist, hat sich als äußerst vorteilhaft auch die Tatsache erwiesen, dass die sonst in den Estern üblicherweise enthaltenen Alkohole nun nicht mehr die Zwischen- und Endprodukte verunreinigen und auch nicht mehr entsorgt werden müssen. Vor allen Dingen weitere Nebenprodukte, wie sie bspw. durch eine unerwünschte Chlorierung oder Alkylierung aufgrund vorhandener Alkoholspuren entstehen, können vermieden werden.

Diese Vorteile des neuen Verfahrens, vor allem aber die Überwindung des Vorurteils, dass Säuren unter ammonolytischen Bedingungen nicht als direkte Ausgangsverbindungen für die Herstellung von Amino- oder Hydroxybenzonitrilen eingesetzt werden können, waren so nicht zu erwarten.

Wie den Angaben zum Herstellungsschritt 1) zu entnehmen ist, handelt es sich diesbezüglich beim Verfahren gemäß Erfindung um eine typische Ammonolyse. Diese als Herstellungsschritt 1), aber auch der Aufreinigungsschritt 2) sind nicht auf bestimmte Temperaturbereiche festgelegt, jedoch hat es sich als günstig erwiesen, den Herstellungsschritt 1) bei Temperaturen zwischen 340 und 450 °C und besonders bevorzugt zwischen 380 und 420 °C durchzuführen.

Ein wesentlicher Faktor für das Gelingen des beanspruchten Kombinationsverfahrens ist in der Verwendung geeigneter Katalysatoren zu sehen. Diesbezüglich wird insbesondere auf die deutsche Offenlegungsschrift 195 20 491 hingewiesen, in der die auch im vorliegenden erfindungsgemäßen Verfahren bevorzugt verwendeten dotierten Borphosphat-Trägerkatalysatoren genauer beschrieben sind.

So wird für das vorliegende Verfahren generell ein Phosphat-haltiger Trägerkatalysator eingesetzt, der mit Übergangsmetall-Verbindungen der 5., 6., 12. und 14. Gruppe des Periodensystems der Elemente oder deren Kombinationen dotiert wurde und der eine spezifische Oberfläche von mindestens 300 m²/g aufweist. Vorzugsweise kann auch ein Trägerkatalysator eingesetzt werden, der zusätzlich mit Bor dotiert ist. In diesem Fall sollte der Anteil an Borphosphat zwischen 0,01 und 15 Gew.-% betragen, wobei Anteile zwischen 0,5 und 5 Gew.-%, jeweils bezogen auf das Gewicht des Trägermaterials, als besonders geeignet anzusehen sind.

Hinsichtlich der Herstellung eines solchen Borphosphat-Trägerkatalysators unterliegt die vorliegende Erfindung keinerlei Beschränkung. Allerdings haben sich Trägerkatalysatoren als besonders gut geeignet herausgestellt, die durch Behandlung des Trägermaterials mit wässrigen Lösungen von 0,01 bis 15 Gew.-% Phosphorsäure, 0,01 bis 15 Gew.-% Borsäure und 0,01 bis 5 Gew.-% Salzen von Übergangsmetallen der 5., 6., 12. oder 14. Gruppe des Periodensystems der Elemente oder Kombinationen solcher Salze hergestellt wurden. Diese Salze der Übergangsmetalle werden in der vorgelegten Lösung angelöst und nachfolgend ca. 1 Stunde bei Raumtemperatur gerührt. Vorzugsweise werden hierfür Salze eingesetzt, die aus Kationen der jeweiligen Elemente und aus dem Anion Borat, Chlorid, Phosphat oder Sulfat bestehen. Möglich ist allerdings auch, die Salze aus den Anionen der jeweiligen Elemente und Ammonium als Kation zu bilden.

Als bevorzugte Kationen für den Trägerkatalysator sind solche von Vanadium, Niob, Tantal, Chrom, Molybdän, Wolfram, Cadmium, Quecksilber, Germanium, Zinn, Blei oder Zink zu sehen, wobei natürlich auch wieder Kombinationen entsprechender Salze eingesetzt werden können.

Zu den so vorgelegten Lösungen mit den entsprechenden Kationen und Anionen werden üblicherweise 20 bis 80 Gew.-% eines Trägermaterials zugegeben, das aus der Gruppe Siliciumdioxid, Kieselgel, Aluminiumoxid, Titanoxid oder Zirkonoxid bzw. Mischungen davon ausgewählt wird. Dieses Trägermaterial sollte eine spezifische Oberfläche von mindestens 400 m²/g aufweisen. Das Wasser wird anschließend verdampft, was auch unter Vakuum erfolgen kann, und der erhaltene Rohkatalysator wird maximal 3 Stunden bei Temperaturen zwischen 100 °C und 500 °C getrocknet, wobei sich das Überleiten eines schwachen Luftstromes als günstig erwiesen hat. Als besonders geeignete Trocknungstemperatur hat sich der Bereich zwischen 140 °C und 160 °C herausgestellt. Der so herstellbare Trägerkatalysator weist erfindungsgemäß eine spezifische Oberfläche auf, die mehr als 400 m²/g beträgt. Als besonders vorteilhaft sind spezifische Oberflächen anzusehen, die > 500 m²/g, > 600 m²/g und insbesondere 750 m²/g betragen. Der Porendurchmesser des Trägerkatalysators sollte vorzugsweise zwischen 0,4 nm und 25 nm und insbesondere zwischen 0,5 nm und 15 nm liegen.

Hinsichtlich des eigentlichen Herstellungsschrittes 1) für die Amino- oder Hydroxybenzonitrile sollte im Rahmen der vorliegenden Erfindung ein Reaktionsgas(-gemisch) eingesetzt werden, das sauerstofffrei und/oder überwiegend Ammoniak-haltig ist. Dieses Reaktionsgas(-gemisch) ist für das erfindungsgemäße Verfahren von besonderer Bedeutung, da es als Inertgas eingesetzt werden und/oder auch als Trägergas fungieren kann.

Für den Herstellungsschritt 1), der ausschließlich in der Gasphase durchgeführt wird, kann es zusätzlich von Vorteil sein, wenn die Amino- oder Hydroxybenzoesäure-Verbindung in das Reaktionsgas(-gemisch) und/oder das Katalysatorbett eingebracht wird, was auch durch Ein- oder Aufdüsen auf den Katalysator erfolgen kann und das die vorliegende Erfindung ebenfalls berücksichtigt. Die jeweiligen Ausgangsverbindungen können vorzugsweise als Säure oder Säureamid (wässrige Lösung eines Ammonium-Salzes), als Schmelze oder in Form eines Feststoffes auf den Katalysator aufgebracht werden.

Wie bereits ausdrücklich erwähnt, gelingt der Herstellungsschritt 1) des erfindungsgemäßen Kombinationsverfahrens, ohne dass sich die eingesetzten Säure-Verbindungen in der Gasphase zersetzen, was möglicherweise auf einen stabilisierenden Effekt des eingesetzten Ammoniakgases zurückzuführen ist, wodurch die bei Säureeinsatz sonst beobachteten Decarboxylierungsneigungen zurückgedrängt bzw. gänzlich vermieden werden. Festzuhalten ist, dass sich das Produkt beim Verfahren gemäß vorliegender Erfindung lediglich beim Verlassen des Reaktors in der Gasphase befindet, weshalb nachhaltige Effekte, wie z. B. Trimerisierungsreaktionen vermieden werden. Festzustellen ist auch, dass das aus dem Herstellungsschritt 1) erhältliche Produkt normalerweise in Form eines weißen bis hellbraunen und flockigen, schuppigen Produktes anfällt, welches leicht zu gewinnen und dem nachfolgenden zweistufigen Aufreinigungsschritt 2) zuzuführen ist.

Als geeignete Temperaturen für den Aufreinigungsschritt 2) des erfindungsgemäßen Verfahrens hat sich ein Bereich zwischen - 20 °C und 100 °C als vorteilhaft erwiesen, wobei sich die Bereiche zwischen 0 °C und 60 °C und insbesondere zwischen 2 °C und 7 °C für bestimmte Verfahrensvarianten als besonders empfehlenswert erwiesen haben.

Wie bereits betont, sollte zwar das im Herstellungsschritt 1) verwendete Reaktionsgas(-gemisch) im Wesentlichen sauerstofffrei sein, jedoch kann es erforderlich sein, den Aufreinigungsschritt 2) zur Vermeidung etwaiger Störungen zusätzlich unter Inertgas-Bedingungen und hierbei vorzugsweise in einer Stickstoffatmosphäre durchzuführen.

Hinsichtlich des Aufreinigungsschrittes 2) ist als erfindungswesentlich festzuhalten, dass zunächst das aus dem Herstellungsschritt 1) erhaltene gasförmige Gemisch in eine wässrige, ammoniumhaltige Suspension überführt wird. Hierfür empfiehlt die vorliegende Erfindung die Anwendung einer Quenche, die vorzugsweise mit Wasser oder einem ammoniakalischen Wasser durchgeführt wird. Das ammoniakalische Wasser, welches natürlich einen basischen Charakter aufweist, wird eingesetzt, falls ein Produkt gequencht werden soll, das in fester Form vorliegt.

Um für den Aufreinigungsschritt 2.2 tatsächlich eine Suspension zu erhalten, empfiehlt es sich, die aus dem Herstellungsschritt 1) erhaltenen Produkte zunächst abzukühlen und den erhaltenen Feststoff anschließend mit einem ammoniakalischen Wasser aufzunehmen.

Unabhängig davon, ob die wässrige basische Suspension mit Hilfe einer Quenche oder durch Abkühlen und eine sich anschließende Aufnahme erhalten wird, kann es für die Produktqualität im Rahmen des beanspruchten Verfahrens notwendig sein, eine wässrige, basische Suspension in hochkonzentrierter Form vorzulegen, in der die Nebenprodukte in gelöster Form vorliegen.

Aus diesen Suspensionen lassen sich nämlich das Endprodukt und die Nebenprodukte besonders effektiv und mit geringem Aufwand abtrennen, da die jeweiligen Produktgruppen bereits in unterschiedlichen Aggregatzuständen vorliegen.

Der letzte Teilschritt des Aufreinigungsschrittes 2) besteht darin, dass aus der aus Verfahrensstufe 2.1) erhaltenen wässrigen, basischen Suspension das im Feststoff vorliegende Produkt freigesetzt wird. Für diese Freisetzung sieht das erfindungsgemäße Verfahren saure Bedingungen vor, wodurch das vorzugsweise im Feststoff als Ammoniumsalz vorliegende Produkt in reiner Form erhalten wird.

Bzgl. dieser Freisetzung sind erfindungsgemäß drei Varianten möglich, die im Bedarfsfall auch untereinander kombiniert werden können.

Variante 1 besteht darin, dass die Freisetzung mit Hilfe einer Filtration und unter Säurezugabe erfolgt oder aber alternativ durch Austreiben von Ammoniak und das sich anschließende Einleiten der Säurekomponente. Zur Gewährleistung der sauren Bedingungen empfiehlt sich die Zugabe einer Säure, wofür sich Mineralsäuren und insbesondere Salzsäure als besonders geeignet gezeigt haben. Möglich ist allerdings auch das Einleiten eines sauren Gases, wie z. B. CO₂. Abschließend können sich in Abhängigkeit vom angestrebten Produkt bzw. dessen Reinheit noch mindestens ein Waschschritt sowie mindestens ein Trocknungsschritt anschließen.

Die zweite Variante zur Freisetzung des Produktes unter sauren Bedingungen besteht darin, eine Reaktivdestillation durchzuführen. Dabei wird üblicherweise erst das gebundene Ammonium als Ammoniak freigesetzt und abgetrennt, wodurch das Produkt schließlich in reiner Form gewonnen wird.

Als dritte Alternative zur Freisetzung des vorzugsweise im Feststoff als Ammoniumsalz vorliegenden Produktes wird die Abspaltung in einem relativ breiten Temperaturbereich zwischen 0 °C und 100 °C vorgeschlagen. Empfehlenswert ist bei dieser Art der Behandlung der Produkt-haltigen Suspension insbesondere ein Temperaturbereich, der zwischen 20 °C und 80 ° C liegt, wobei auch Vakuumbedingungen geeignet sein können. Druckbereiche zwischen 0,1 mbar und 1 bar und insbesondere zwischen 500 mbar und 800 mbar sind als besonders bevorzugt anzusehen. Diese im Wesentlichen thermische Abspaltung kann auch in Gegenwart eines organischen Lösemittels, wie z. B. DMF, durchgeführt werden. Diese letztgenannte Alternative ist die einzige Ausnahme, bei der im beanspruchten Verfahren ein organisches Lösemittel verwendet wird.

Als treibende Kraft für diese Abspaltung kann der Solvatationsdruck angesehen werden, wie er allgemein bei thermischen Behandlungen organischer Lösungen auftritt. In diesen Fällen sind Temperaturbereiche zwischen 20 und 80 °C und Drücke zwischen 1 mbar und 1 bar zu empfehlen.

Unter diesen Bedingungen erhält man eine klare Lösung als leichte Fraktion, die abgetrennt wird, und eine zweite Fraktion, die das gewünschte Nitril im Lösemittel gelöst enthält.

Mit dem erfindungsgemäßen Verfahren, bestehend aus dem Herstellungsschritt 1) und dem Aufreinigungsschritt 2), lassen sich insbesondere Benzonitrile mit deprotonierbaren Substituenten, wie sie bspw. Hydroxygruppen darstellen, erhalten, wobei vor allem das 2-Hydroxybenzonitril und das 4-Hydroxybenzonitril, aber auch das 2,4-Dihydroxybenzonitril im Vordergrund stehen. Diese Produkte zeichnen sich durch eine einzigartige Produktqualität aus, da sie praktisch keinerlei Verunreinigungen durch organische Lösemittel und alkylierte Nebenprodukte aufweisen, was insbesondere darin begründet ist, dass sie durch eine direkte Ammonolyse aus deren Säuren erhältlich sind. Bei den Produkten handelt es sich somit typischerweise um lösemittelfreie Amino- und Hydroxybenzonitrile. Weitere Vorteile des erfindungsgemäßen Verfahrens sind darin zu sehen, dass jeweils die wirtschaftlichsten Rohstoffe eingesetzt werden und dass die bereits angesprochenen hohen Ausbeuten in der Regel durch die nur sehr geringen Decarboxydierungsverluste erzielt werden. Dabei wird der eingesetzte Ammoniak durch relativ geringe Ausschleusraten, verbunden mit einer geringen CO₂-Bildung effizient ausgenutzt. Da durch die spezielle Verfahrensführung eine saure Salzspaltung vermieden wird, werden bei der großtechnischen Anwendung des vorgeschlagenen Verfahrens keine Sonderwerkstoffe nötig, sondern es können in der Regel günstige Werkstoffe eingesetzt werden. Hinzu kommt, dass Chlorid-haltige Abwässer vermieden werden, und dass die Gesamtabwassermenge aufgrund der speziellen Verfahrensführung deutlich reduziert werden kann.

Die nachfolgenden Beispiele verdeutlichen die Vorteile des erfindungsgemäßen Verfahrens.

### Beispiele

Herstellung und Aufreinigung von 2-Hydroxybenzonitril

### 1) Herstellungsschritt

Für die nachfolgend beschriebene Herstellung wurde ein Borphosphat-Trägerkatalysator eingesetzt, der mit folgendem Verfahren erhalten wurde:
5,88 g einer 85 %igen Orthophosphorsäure und 3,33 g einer 99,8 %igen Borsäure wurden in 500 g destilliertem Wasser gelöst. Nach zusätzlichem Lösen von 3,6 g Zinksulfat wurde diese Lösung eine Stunde bei Raumtemperatur gerührt und anschließend wurden 500 g Kieselgel zugegeben, dessen Oberfläche > 400 m²/g betrug; der Porendurchmesser des Kieselgels lag zwischen 0,4 nm und 25 nm. Anschließend wurde bei 60 °C unter vermindertem Druck bis zur Trockne eingedampft und abschließend drei Stunden lang auf 150 °C unter Überleiten eines schwachen Luftstromes erhitzt. Nach dem Abkühlen konnte der so erhaltene Trägerkatalysator wie nachfolgend beschrieben eingesetzt werden.

In den Reaktor wurden 50 ml des Zink-dotierten Borphosphat-Trägerkatalysators eingebracht. Anschließend wurden bei Temperaturen von 340 °C 2,1 mol/h Ammoniakgas und 350 mmol 2-Hydroxybenzoesäure mit einer Geschwindigkeit von 70 mmol/h in den sauerstofffreien Gasraum eingedüst. In einem Abscheider wurde der ausgetretene Gasstrom abgekühlt, worauf sich das Reaktionsprodukt als weißer, flockiger Feststoff abschied.

### 2) Aufreinigungsschritt

500 g eines gemäß Herstellungsschritt 1) erhaltenen Rohproduktes wurden vorgelegt und mit 1.500 ml wässrigem Ammoniak (als 32 %ige Lösung) vermischt und anschließend bei Raumtemperatur 60 Minuten lang gerührt.

Die aus diesem Quenchvorgang erhaltene basische Suspension wurde abgenutscht und der Filterkuchen zweimal mit je 187,5 ml 32 %igem ammoniakalischen Wasser gewaschen. Anschließend wurde der Filterkuchen 20 Minuten trocken gesaugt und mit 1.250 ml 15 %iger HCl versetzt. Unter diesen sauren Bedingungen wurde die Suspension 45 Minuten lang bei Raumtemperatur gerührt, der Feststoff abgenutscht und zweimal mit je 187,5 ml einer 15 %igen HCl gewaschen. Dann wurde der Feststoff getrocknet und mit 360 ml demineralisiertem Wasser versetzt, 15 Minuten gerührt und abgesaugt. Der so erhaltene Feststoff wurde im Vakuum über Nacht bei 50 °C getrocknet.

Durch das Herstellungsverfahren und den sich anschließenden Aufreinigungsschritt wurde 2-Hydroxybenzonitril in Ausbeuten von 75 % der Theorie mit einer Reinheit von 98,9 % erhalten.

## Patentansprüche

1. Verfahren zur Herstellung von Amino- und Hydroxybenzonitrilen der allgemeinen Formel (I) 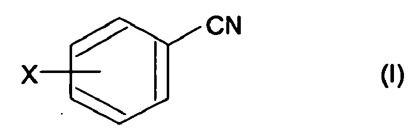 in der X für mindestens eine Amino- oder Hydroxygruppe steht, durch Umsetzung einer Amino- oder Hydroxybenzoesäure der allgemeinen Formel (II) 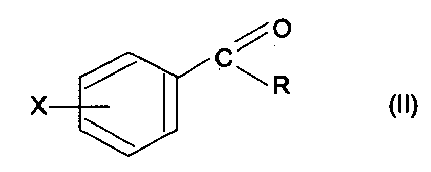 wobei R = -OH, -NH₂ und X die oben genannte Bedeutung besitzt, mit Ammoniak in Gegenwart eines Phosphor-haltigen Trägerkatalysators bei Temperaturen zwischen 250 und 500 °C, **dadurch gekennzeichnet, dass** der Herstellungsschritt 1) in einem Reaktionsgas(-gemisch) und ohne Beteiligung eines organischen Lösemittels durchgeführt wird und sich anschließend ein mindestens zweistufiger Aufreinigungsschritt 2) anschließt, bei dem 2.1) das aus dem Herstellungsschritt 1) erhaltene gasförmige Gemisch oder feste Reaktionsprodukt in eine wässrige, ammoniumhaltige Suspension überführt wird und 2.2)
a) die Freisetzung des im Feststoff als Ammoniumsalz vorliegenden Produktes mithilfe einer Filtration unter Säurezugabe oder durch Austreiben von Ammoniak und ein sich anschließendes Einleiten der Säurekomponente erfolgt, oder
b) die Freisetzung des im Feststoff als Ammoniumsalz vorliegenden Produktes durch eine Reaktivdestillation vorgenommen wird, oder
c) die Freisetzung des im Feststoff als Ammoniumsalz vorliegenden Produktes durch eine Abspaltung bei Temperaturen von 0 bis 100°C und in Gegenwart eines organischen Lösemittels durchgeführt wird.

2. Verfahren nach Anspruch 1, **dadurch gekennzeichnet, dass** das Verfahren bei Temperaturen zwischen 340 und 450 °C durchgeführt wird.

3. Verfahren nach einem der Ansprüche 1 oder 2, **dadurch gekennzeichnet, dass** der Katalysator mit Übergangsmetall-Verbindungen der 5., 6., 12. und 14. Gruppe des Periodensystems der Elemente oder deren Kombinationen dotiert wurde und dass seine spezifische Oberfläche mindestens 300 m²/g beträgt.

4. Verfahren nach einem der Ansprüche 1 bis 3, **dadurch gekennzeichnet, dass** ein Trägerkatalysator eingesetzt wird, der zusätzlich mit Bor dotiert ist.

5. Verfahren nach einem der Ansprüche 1 bis 4, **dadurch gekennzeichnet, dass** man einen Borphosphat Trägerkatalysator verwendet, der durch Behandlung des Trägermaterials mit wässrigen Lösungen von 0,01 bis 15 Gew.-% Phosphorsäure, 0,01 bis 15 Gew.-% Borsäure und 0,01 bis 5 Gew.-% Salzen von Übergangsmetallen der 5., 6., 12. oder 14. Gruppe des Periodensystems der Elemente oder Kombinationen solcher Salze hergestellt wurde.

6. Verfahren nach einem der Ansprüche 1 bis 5, **dadurch gekennzeichnet, dass** das Trägermaterial aus Siliciumdioxid, Kieselgel, Aluminiumoxid, Titanoxid oder Zirkonoxid bzw. Mischungen davon besteht.

7. Verfahren nach einem der Ansprüche 1 bis 6, **dadurch gekennzeichnet, dass** Salze der Elemente Vanadium, Niob, Tantal, Chrom, Molybdän, Wolfram, Cadmium, Quecksilber, Germanium, Zinn, Blei oder Zink bzw. Kombinationen dieser Salze eingesetzt wurden.

8. Verfahren nach Anspruch 7, **dadurch gekennzeichnet, dass** die Salze aus Kationen der jeweiligen Elemente und aus dem Anion Borat, Chlorid, Phosphat oder Sulfat bestanden.

9. Verfahren nach Anspruch 7**, dadurch gekennzeichnet, dass** die Salze aus Anionen der jeweiligen Elemente und Ammonium als Kation bestanden.

10. Verfahren nach einem der Ansprüche 1 bis 9, **dadurch gekennzeichnet, dass** die spezifische Oberfläche des dotierten Trägerkatalysators > 400 m²/g beträgt.

11. Verfahren nach einem der Ansprüche 1 bis 10, **dadurch gekennzeichnet, dass** der dotierte Borphosphat-Trägerkatalysator einen Porendurchmesser zwischen 0,4 und 25 nm besitzt.

12. Verfahren nach einem der Ansprüche 1 bis 11, **dadurch gekennzeichnet, dass** der dotierte Trägerkatalysator bis zu 3 Stunden bei Temperaturen zwischen 100 und 500 °C getrocknet wurde.

13. Verfahren nach einem der Ansprüche 1 bis 12, **dadurch gekennzeichnet, dass** das Reaktionsgas(-gemisch) sauerstofffrei und Ammoniak-haltig ist.

14. Verfahren nach einem der Ansprüche 1 bis 13, **dadurch gekennzeichnet, dass** die Amino- oder Hydroxybenzoesäure in das Reaktionsgas(-gemisch) und/oder das Katalysatorbett eingebracht wird, was vorzugsweise als Schmelze, Feststoff oder wässrige Lösung des Ammonium-Salzes geschieht.

15. Verfahren nach einem der Ansprüche 1 bis 14, **dadurch gekennzeichnet, dass** der Aufreinigungsschritt 2) bei Temperaturen zwischen - 20 °C und 100 °C durchgeführt wird.

16. Verfahren nach einem der Ansprüche 1 bis 15, **dadurch gekennzeichnet, dass** der Aufreinigungsschritt 2) unter Inertgas-Bedingungen durchgeführt wird.

17. Verfahren nach einem der Ansprüche 1 bis 16, **dadurch gekennzeichnet, dass** im Aufreinigungsschritt 2) die wässrige, ammoniumhaltige Suspension mit Hilfe einer Quenche erhalten wird, die mit Wasser oder einem ammoniakalischen Wasser durchgeführt wird.

18. Verfahren nach einem der Ansprüche 1 bis16, **dadurch gekennzeichnet, dass** im Aufreinigungsschritt 2) die wässrige, ammoniumhaltige Suspension durch Abkühlen des aus dem Herstellungsschritt 1) erhaltenen Produkts und eine sich anschließende Aufnahme mit einem ammoniakalischen Wasser gewonnen wird.

19. Verfahren nach einem der Ansprüche 1 bis 18, **dadurch gekennzeichnet, dass** die wässrige, ammoniumhaltige Suspension in hochkonzentrierter Form vorliegt und die Nebenprodukte in gelöster Form enthält.

20. Verfahren nach einem der Ansprüche 1 bis 19, **dadurch gekennzeichnet, dass** die sauren Bedingungen durch Zugabe einer Säure, oder durch Einleiten eines sauren Gases eingestellt wird.

21. Verfahren nach einem der Ansprüche 1 bis 20, **dadurch gekennzeichnet, dass** abschließend mindestens ein Waschschritt sowie mindestens ein Trocknungsschritt erfolgen.

## Claims

1. Process for producing amino- and hydroxybenzonitriles of the general formula (I) 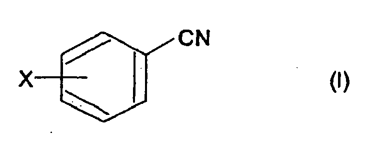 in which X represents at least one amino or hydroxy group, by reacting an amino- or hydroxybenzoic acid of the general formula (II) 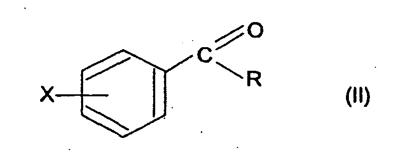 in which R = -OH or -NH₂ and X has the above-mentioned meaning, with ammonia in the presence of a supported catalyst containing phosphorus at temperatures between 250 and 500°C, **characterized in that** the production step 1) is carried out in a reaction gas (mixture) and without participation of an organic solvent and is followed by an at least two-stage purification step 2) in which 2.1) the gaseous mixture or solid reaction product obtained from production step 1) is converted into an aqueous, ammonium-containing suspension and 2.2)
a) the product present in the solid as an ammonium salt is released with the aid of a filtration with addition of acid or by expulsion of ammonia and subsequently introducing the acid component, or
b) the product present in the solid as an ammonium salt is released by a reactive distillation, or
c) the product present in the solid as an ammonium salt is released by elimination at temperatures of 0 to 100°C and in the presence of an organic solvent.

2. Process according to claim 1, **characterized in that** the process is carried out at temperatures between 340 and 450°C.

3. Process according to one of the claims 1 or 2, **characterized in that** the catalyst has been doped with transition metal compounds of groups 5, 6, 12 and 14 of the periodic system of the elements or combinations thereof and that its specific surface area is at least 300 m²/g.

4. Process according to one of the claims 1 to 3, **characterized in that** a supported catalyst is used which has been additionally doped with boron.

5. Process according to one of the claims 1 to 4, **characterized in that** a boron phosphate supported catalyst is used which has been prepared by treating the support material with aqueous solutions of 0.01 to 15 % by weight phosphoric acid, 0.01 to 15 % by weight boric acid and 0.01 to 5 % by weight salts of transition metals of group 5, 6, 12 or 14 of the periodic system of the elements or combinations of such salts.

6. Process according to one of the claims 1 to 5, **characterized in that** the support material consists of silicon dioxide, silica gel, aluminium oxide, titanium oxide or zirconium oxide or mixtures thereof.

7. Process according to one of the claims 1 to 6, **characterized in that** salts of the elements vanadium, niobium, tantalum, chromium, molybdenum, tungsten, cadmium, mercury, germanium, tin, lead or zinc or combinations of these salts have been used.

8. Process according to claim 7, **characterized in that** the salts consisted of cations of the respective elements and of the borate, chloride, phosphate or sulphate anion.

9. Process according to claim 7, **characterized in that** the salts consisted of anions of the respective elements and ammonium as the cation.

10. Process according to one of the claims 1 to 9, **characterized in that** the specific surface area of the doped supported catalyst is > 400 m²/g.

11. Process according to one of the claims 1 to 10, **characterized in that** the doped boron phosphate supported catalyst has a pore diameter between 0.4 and 25 nm.

12. Process according to one of the claims 1 to 11, **characterized in that** the doped supported catalyst has been dried at temperatures between 100 and 500°C for up to three hours.

13. Process according to one of the claims 1 to 12, **characterized in that** the reaction gas (mixture) is oxygen-free and contains ammonia.

14. Process according to one of the claims 1 to 13, **characterized in that** the amino- or hydroxybenzoic acid is introduced into the reaction gas (mixture) and/or the catalyst bed which preferably occurs as a melt, solid or aqueous solution of the ammonium salt.

15. Process according to one of the claims 1 to 14, **characterized in that** the purification step 2) is carried out at temperatures between -20°C and 100°C.

16. Process according to one of the claims 1 to 15, **characterized in that** the purification step 2) is carried out under inert gas conditions.

17. Process according to one of the claims 1 to 16, **characterized in that** in the purification step 2) the aqueous, ammonium-containing suspension is obtained with the aid of a quench which is carried out with water or ammoniacal water.

18. Process according to one of the claims 1 to 16, **characterized in that** in purification step 2) the aqueous, ammonium-containing suspension is obtained by cooling the product obtained from production step 1) and a subsequent absorption with ammoniacal water.

19. Process according to one of the claims 1 to 18, **characterized in that** the aqueous, ammonium-containing suspension is present in a highly concentrated form and contains the by-products in a dissolved form.

20. Process according to one of the claims 1 to 19, **characterized in that** the acidic conditions are established by adding an acid or by passing in an acidic gas.

21. Process according to one of the claims 1 to 20, **characterized in that** finally at least one washing step and at least one drying step are carried out.

## Revendications

1. Procédé de préparation d'amino- et d'hydroxybenzonitriles de la formule générale (I) 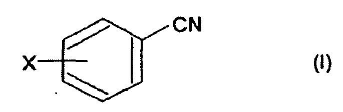 dans laquelle X représente un groupe amine ou hydroxyle, par réaction d'un acide amino- ou hydroxybenzoïque de la formule générale (II) 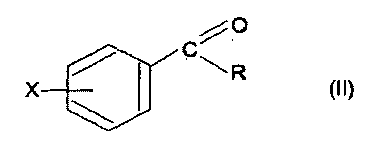 dans laquelle R = -OH, -NH₂, et X a la signification formulée ci-dessus, avec de l'ammoniac en présence d'un catalyseur phosphoré sur support à des températures comprises entre 250 et 500 °C, **caractérisé en ce que** l'étape de préparation 1) est effectuée dans un (mélange de) gaz réactionnel(s) et sans contribution d'un solvant organique et **en ce qu'**ensuite, il suit une étape de purification 2) au moins biétagée, lors de laquelle 2.1) le mélange gazeux ou le produit de réaction solide obtenu à l'issue de l'étape de préparation 1) est converti en une suspension aqueuse contenant des ions ammoniums et 2.2)
a) la libération du produit présent dans le solide sous forme de sel d'ammonium s'effectue à l'aide d'une filtration avec addition d'acide ou par élimination d'ammoniac suivie de l'introduction du composant acide, ou
b) la libération du produit présent dans le solide sous forme de sel d'ammonium est réalisée par une distillation réactive, ou
c) la libération du produit présent dans le solide sous forme de sel d'ammonium est effectuée par une élimination à des températures de 0 à 100 °C en présence d'un solvant organique.

2. Procédé selon la revendication 1, **caractérisé en ce que** le procédé est effectué à des températures comprises entre 340 et 450 °C.

3. Procédé selon l'une quelconque des revendications 1 ou 2, **caractérisé en ce que** le catalyseur a été dopé avec des composés des métaux de transition des 5^{e}, 6^{e}, 12^{e} et 14^{e} groupes du système périodique des éléments, ou d'une combinaison de ceux-ci, et **en ce que** sa surface spécifique est d'au moins 300 m²/g.

4. Procédé selon l'une quelconque des revendications 1 à 3, **caractérisé en ce que** l'on utilise un catalyseur supporté qui est dopé en plus avec du bore.

5. Procédé selon l'une quelconque des revendications 1 à 4, **caractérisé en ce que** l'on utilise un catalyseur supporté de phosphate de bore qui a été fabriqué par traitement du support avec des solutions aqueuses contenant de 0,01 à 15 % en poids d'acide phosphorique, de 0,01 à 15 % en poids d'acide borique et de 0,01 à 5 % en poids de sels de métaux de transition des 5^{e}, 6^{e}, 12^{e} et 14^{e} groupes du système périodique des éléments, ou d'une combinaison de tels sels.

6. Procédé selon l'une quelconque des revendications 1 à 5, **caractérisé en ce que** le matériau support est composé de dioxyde de silicium, de gel de silice, d'oxyde d'aluminium, d'oxyde de titane ou d'oxyde de zirconium, ou de mélanges de ceux-ci.

7. Procédé selon l'une quelconque des revendications 1 à 6, **caractérisé en ce que** l'on a utilisé des sels des éléments vanadium, niobium, tantale, chrome, molybdène, tungstène, cadmium, mercure, germanium, étain, plomb ou zinc, ou une combinaison de ces sels.

8. Procédé selon la revendication 7, **caractérisé en ce que** les sels étaient constitués des cations de ces éléments et d'un anion borate, chlorure, phosphate ou sulfate.

9. Procédé selon la revendication 7, **caractérisé en ce que** les sels étaient constitués des anions de ces éléments respectifs et d'ammonium en tant que cation.

10. Procédé selon l'une quelconque des revendications 1 à 9, **caractérisé en ce que** la surface spécifique du catalyseur supporté dopé est >400 m²/g.

11. Procédé selon l'une quelconque des revendications 1 à 10, **caractérisé en ce que** le catalyseur supporté dopé à base de phosphate de bore a un diamètre de pore compris entre 0,4 et 25 nm.

12. Procédé selon l'une quelconque des revendications 1 à 11, **caractérisé en ce que** le catalyseur supporté dopé a été séché jusqu'à 3 heures à des températures comprises entre 100 et 500 °C.

13. Procédé selon l'une quelconque des revendications 1 à 12, **caractérisé en ce que** le (mélange de) gaz réactionnel(s) est exempt d'oxygène et contient de l'ammoniac.

14. Procédé selon l'une quelconque des revendications 1 à 13, **caractérisé en ce que** l'acide amino- ou hydroxybenzoïque est introduit dans le (mélange de) gaz réactionnel(s) et/ou dans le lit de catalyseur, ce qui s'effectue de préférence sous forme de matière fondue, de solide ou de solution aqueuse du sel d'ammonium.

15. Procédé selon l'une quelconque des revendications 1 à 14, **caractérisé en ce que** l'étape de purification 2) est réalisée à des températures comprises entre -20°C et 100 °C.

16. Procédé selon l'une quelconque des revendications 1 à 15, **caractérisé en ce que** l'étape de purification 2) est réalisée sous atmosphère de gaz inerte.

17. Procédé selon l'une quelconque des revendications 1 à 16, **caractérisé en ce que** dans l'étape de purification 2), la suspension aqueuse ammoniacale est obtenue à l'aide d'un brusque refroidissement, réalisée avec de l'eau ou de l'eau ammoniacale.

18. Procédé selon l'une quelconque des revendications 1 à 16, **caractérisé en ce que,** dans l'étape de purification 2), la suspension aqueuse ammoniacale est obtenue par refroidissement du produit obtenu à l'issue de l'étape de production 1), suivi d'une reprise dans de l'eau ammoniacale.

19. Procédé selon l'une quelconque des revendications 1 à 18, **caractérisé en ce que** la suspension aqueuse ammoniacale est présente sous une forme hautement concentrée et contient les produits secondaires sous forme dissoute.

20. Procédé selon l'une quelconque des revendications 1 à 19, **caractérisé en ce que** les conditions acides sont ajustées par addition d'un acide ou par introduction d'un gaz acide.

21. Procédé selon l'une quelconque des revendications 1 à 20, **caractérisé en ce que** l'on fait suivre au moins une étape de lavage ainsi qu'au moins une étape de séchage.
